(19) Europäisches Patentamt
European Patent Office
Office européen des brevets

(11) Publication number: **0 033 068**
**B2**

(12) # NEW EUROPEAN PATENT SPECIFICATION

(45) Date of publication of the new patent specification: **04.03.87**

(51) Int. Cl.$^4$: **C 07 C 17/38**

(21) Application number: **81100080.1**

(22) Date of filing: **08.01.81**

(54) **Process for reducing the concentration of chloride in a system containing ethylenically unsaturated chlorinated hydrocarbon, water and HCl.**

(30) Priority: **28.01.80 US 116190**

(43) Date of publication of application:
**05.08.81 Bulletin 81/31**

(45) Publication of the grant of the patent:
**20.07.83 Bulletin 83/29**

(45) Mention of the opposition decision:
**04.03.87 Bulletin 87/10**

(84) Designated Contracting States:
**DE FR GB**

(56) References cited:
**US-A-2 852 572**
**US-A-3 691 239**
**US-A-3 801 659**

(73) Proprietor: **International Business Machines Corporation**
**Old Orchard Road**
**Armonk, N.Y. 10504 (US)**

(72) Inventor: **Ameen, Joseph George**
**15 Crescent Drive**
**Apalachin New York 13732 (US)**
Inventor: **Joseph, Charles Arnold**
**2736 Robins Street**
**Endwell New York 13760 (US)**
Inventor: **Rivenburgh, Dennis Louis**
**804 Buffalo Street**
**Endicott New York 13760 (US)**
Inventor: **Sissenstein, David William**
**519 Alfred Drive**
**Endwell New York 13760 (US)**

(74) Representative: **Kreidler, Eva-Maria, Dr. rer. nat.**
**Schönaicher Strasse 220**
**D-7030 Böblingen (DE)**

**EP 0 033 068 B2**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for reducing the concentration of chloride in a system which contains HCl, water, and an ethylenically unsaturated chlorinated hydrocarbon. In particular, the present invention is concerned with reducing the chloride content in those compositions which also contain a nitrogen-containing compound of the type that has at least on unshared pair of electrons capable of forming a dative covalent bond. The present invention is especially advantageous in those systems which are used in contact with electronic components such as module substrates, assemblies, and printed circuit boards.

Certain ethylenically unsaturated chlorinated hydrocarbons such as perchloroethylene are employed for cleaning and degreasing electronic components such as module substrates, assemblies, and printed circuit boards. However, at the operating temperature, such hydrocarbons such as perchloroethylene tend to react with water and/or oxygen contaminants to produce, in the case of perchloroethylene, trichloroacetyl chloride which may then subsequently hydrolize to hydrochloric acid.

The presence of chloride can be extremely harmful to the components contacted with the compositions. For instance, chloride can be very detrimental to circuitry resulting in "black fingers" on electrical contacts and hereby contributing to electrolytic corrosion on metalized ceramics. The term "black fingers" refers to corrosion of for instance tin and/or lead on the ceramic resulting in the corresponding chlorides.

In order to remove chloride from such systems, nitrogen containing inhibitors such as diallylamine have been added. The nitrogen compound such as the diallylamine is generally added periodically in order to combine with for instance the intermediate trichloroacetyl chloride and also with the by-product, hydrochloric acid.

Reactions Nos. 2 and 3 hereinbelow illustrate the reactions when adding diallylamine to a perchloroethylene system:

2) 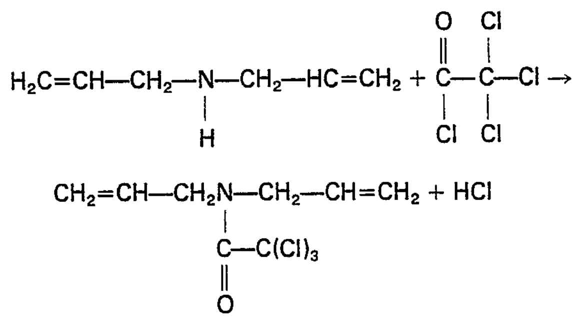

3) 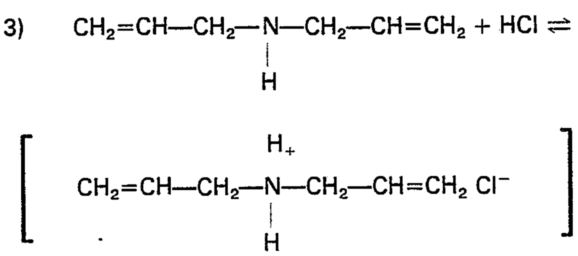

The diallylamine hydrochloride formed from reaction No. 3, illustrated above is an insoluble precipitate in the perchloroethylene and it can thereby be removed in filters. However, it is still present in the system and is in equilibrium therein. If the filters are not changed periodically, more and more of the nitrogen compound (e.g. diallylamine) is needed to control the chloride concentration.

Various different processes have been suggested for purifying liquids such as perchlorethylene to remove certain impurities therein.

For instance, U.S. Patent 3 751 494 is concerned with removing saturated partially-chlorinated hydrocarbons from unsaturated chlorocarbon and chlorinated hydrocarbons including perchlorethylene by treatment with a type 13X molecular sieve. The partially-chlorinated hydrocarbons are delchlorinated and the products of dehydrochlorination are at least partially adsorbed. In the process suggested in U.S. Patent 3,751 494, the action of the molecular sieve on the saturated chlorinated hydrocarbons also results in the production of hydrogen chloride.

U.S. Patent 2 888 495 to Kissling suggests purifying perchloroethylene by contacting it with ion exchange resins in order to reduce the acidity of the perchloroethylene composition. U.S. Patent 3 309 166 to Moncada et. al. suggests an adsorption for purifying various solvents among which is suggested perchloroethylene. U.S. Patent 3 452 110 is exemplary of those patents which suggest filtering processes for purifying solvents including perchloroethylene.

Object of the invention is a process for reducing the concentration of chloride in a system containing an ethylenically unsaturated liquid hydrocarbon, water and HCl.

The object of the invention is achieved with the process comprising the following steps:

a) providing in said system a nitrogen containing compound having a pair of unshared electrons which are capable of forming a dative covalent bond, thereby

b) forming a complex between HCl and said nitrogen containing compound in said system;

c) contacting said system, subsequent to the forming of said complex, with a molecular sieve where, upon contact therewith said complex is broken down into HCl and said nitrogen containing compound, thereby

d) regenerating said nitrogen containing compound and releasing it back into said system, and

e) reducing the HCl concentration in said system to less then 5 ppm through adsorption by the molecular sieve.

The invention makes it possible to provide compositions, after contact with the molecular sieve, which contain less than about 1 ppm of chloride and less than 10 ppm of water.

The inventive process comprises providing in the system with the ethylenically unsaturated chlorinated hydrocarbon a nitrogen-containing compound. The nitrogen-containing compound must have at least one unshared pair of electrons which are capable of forming a dative covalent bond. The system which contains the nitrogen-containing compound is contacted with a molecular sieve to thereby reduce the concentration of chloride in the system. The molecular sieve preferably has an effective pore size of about 0.3 to about 1.0 nm.

A dative bond, also known as a semipolar bond or coordinate covalence, is a covalent bond in which one atom has supplied both electrons thus giving rise to a difference in charge of one electron between two atoms in the same molecule.

The ethylencially unsaturated chlorinated hydrocarbons which are processed according to the present invention are liquid at the conditions of treatment and preferably liquid at normal room temperatures. In addition, the chlorinated hydrocarbons processed according to the present invention are preferably monoethylenically unsaturated . Examples of some ethylenically unsaturated chlorinated hydrocarbons are trichloroethylene, perchloroethylene, hexachloroethylene, and hexachlorobutadine. Ethylenically unsaturated chlorinated hydrocarbons are exemplified by perchloroethylene under conditions of elevated temperatures tend to react with water and/or oxygen contaminants to produce intermediates such as trichloroacetyl chloride in the case of perchloroethylene, which in turn may then hydrolize to hydrochloric acid as exemplified by the following reaction sequence:

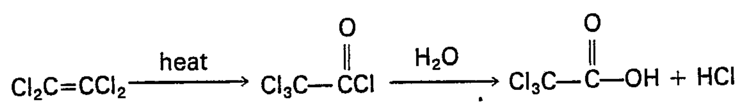


The compositions treated according to the present invention contain water.

The nitrogen-containing compounds which are employed in the process of the present invention must have a pair of unshared electrons which are capable of forming a dative covalent bond. The nitrogen compounds employed in the present invention are generally low molecular weight nitrogen compounds which are liquid and are soluble in the ethylenically unsaturated chlorinated hydrocarbon and include primary and secondary amines. The nitrogen compounds usually have molecular weights up to about 500. Examples of some nitrogen compounds suitable for the present invention are diallylamine, diethylamine, dimethylamine, and diactyl amine. The preferred amine employed according to the present invention is diallylamine. Some of the amines can be represented by the formula

$$RR_1NH$$

wherein R is alkanoyl or aroyl or hydrocarbyl such as alkyl, allyl, cycloalkyl, and aryl; and $R_1$ is H, or alkanoyl or aroyl or hydrocarbyl such as alkyl, allyl, cycloalkyl, and aryl. The number of carbon atoms of the above groups being selected so that the amine is a liquid and has a molecular weight of about 500 or less.

For illustration purposes, using diallylamine and perchloroethylene, the following reactions are believed to occur:

1 1) 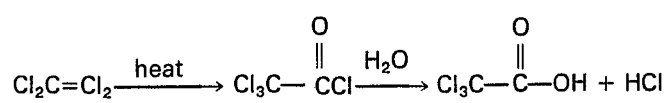


2) 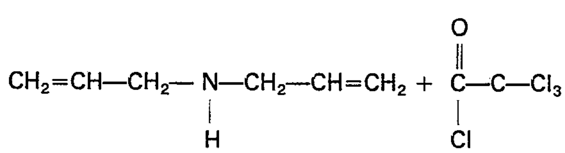

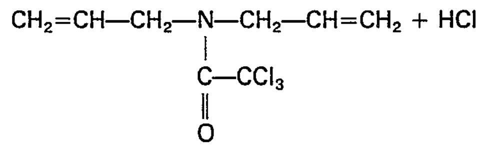

3) 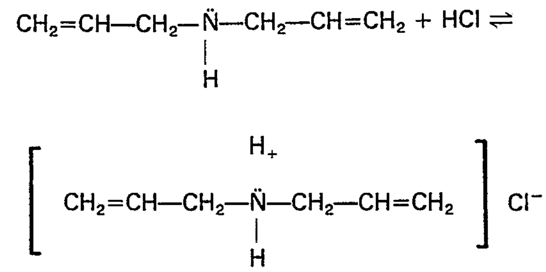

The diallylamine hydrochloride prepared from reaction No. 3 above is an insoluble precipitate in perchloroethylene and is taken out in filters. However, it is still present in the system and is in equilibrium therein. Since there is an equilibrium reaction, chloride will still be present in the system to some extent.

In order to overcome this particular problem, it has been found according to the present invention that contact of the above system with a molecular sieve will result in reduction of the concentration of chloride in the system. The molecular sieves employed preferably have an effective pore size of about 0.3 to about 1.0 nm. Molecular sieves are crystalline metal alumino-silicates.

The molecular sieves are basically a 3-dimensional frameword of $SiO_4$ and $AlO_4$ tetrahedra. The tetrahedra being cross-linked by the sharing of oxygen atoms so that the ratio of oxygen atoms to the total of the aluminum and silicon atoms is equal to 2. The electro valance of the tetrahedra containing aluminum is balanced by the inclusion in the crystal of a cation, for example an alkali or alkaline earth metal ion. One cation may be exchanged for another by ion exchange techniques which are known. The spaces between the tetrahedra are occupied by water molecules prior to dehydration. The dehydration results in crystals interlaced with channels of molecular dimensions that offer very high surface areas for the adsorption of foreign molecules. In addition, the term "molecular sieve" as used in the present disclosure contemplates not only aluminosilicates, but also substances in which the aluminum has been partly or wholly replaced, such as for instance by gallium and/or other metal atoms, and further includes substances in which all or part of the silicon has been replaced, such as for instance by germanium. Titanium and zirconium substitution may also be practiced.

Most molecular sieves, or zeolites as they are also referred to, are prepared or occur naturally in the sodium form, so that sodium cations are associated with the electro negative sites in the crystal structure. However, the molecular sieve may be ion exchanged. Suitable cations of replacement of sodium in the molecular sieve crystal structure include ammonium (decomposable to hydrogen), hydrogen, rare earth metals, alkaline earth metals, and the like. Various suitable ion exchange procedures and cations which may be exchanged into crystal structure are well known to those skilled in the art.

Examples of the naturally occurring crystalline aluminosilicate zeolites which may be used or included in the present invention are faujasite, mordenite, clinoptilote, chabazite, analcite, erionite, as well as levynite, dachiardite, paulingite, noselite, ferriorite, heulandite, scolccite, stibite, harmotome, phillipsite, brewsterite, flarite, datolite, gmelinite, caumnite, leucite, lazurite, scaplite, mesolite, ptholite, nepheline, matrolite, offretite and sodalite.

Examples of the synthetic alumino-silicate zeolites which are useful for carrying out the present invention are Zeolite X, U.S. Patent No. 2,882,244, Zeolite Y, U.S. Patent No. 3,130,007; and Zeolite A, U.S. Patent No. 2,882,243; as well as Zeolite B, U.S. Patent No. 3,008,803; Zeolite D, Canada Patent No. 661,981; Zeolite E, Canada Patent No. 614,495; Zeolite F, U.S. Patent No. 2,996,358; Zeolite H, U.S. Patent No. 3,010,789; Zeolite J, U.S. Patent No. 3,001,869; Zeolite L, Belgium Patent No. 575,177; Zeolite M, U.S. Patent No. 2,995,423; Zeolite O, U.S. Patent No. 3,140,252; Zeolite Q, U.S. Patent No. 2,991,151; Zeolite S, U.S. Patent No. 3,054,657; Zeolite T, U.S. Patent No. 2,950,962; Zeolite W, U.S. Patent No. 3,012,853; Zeolite Z, Canada Patent No. 614,495; and Zeolite Omega, Patent No. 817,915. Also ZK—4HJ, alpha beta and ZSM-type zeolites are useful. Moreover, the zeolites described in U.S. Patents Nos. 3,140,249, 3,140,253, 3,044,482 and 4,137,151 are also useful, the disclosure of said patents being incoporated herein by reference.

The preferred molecular sieves employed according to the present invention are those commercially available from Union Carbide under the trade designations Lindy Molecular sieves types 3A, 4A, 5A and 13X and most preferably the so-called acid resistant sieves, types AW300 and AW500. The effective pore size of these preferred molecular sieves are as follows:

| Type | Pore size |
|---|---|
| 3A | 0.3 nm |
| 4A | 0.4 nm |
| 5A | 0.5 nm |
| 13X | 1.0 nm |
| AW300 | 0.4 nm |
| AW500 | 0.5 nm |

Such are available as pellets of about 0.32 cm or 0.16 cm diameter. A discussion of the acid resistant molecular sieve Types SW—300 and AW—500 can be found in the Oil and Gas Journal, December 2, 1963, A Report On Acid-Resistant Molecular Sieve Types AW—300 and AW—500 by J. J. Collins, disclosures which is incorporated here by reference. In addition, a discussion of Lindy molecular sieve type 3A, Lindy molecular sieve type 4A, Lindy molecular sieve type 5A, and Lindy molecular sieve type 13X can be found in Lindy molecular sieves adsorbent data circulars F—21B, F—3996, F—37, and F—23A resepectively from Union Carbide Corporation. In addition, the preferred molecular sieves employed according to the present invention are alkali metal aluminosilicates synthetic molecular sieves.

The molecular sieves employed adsorb onto the sieve some nitrogen compound such as the diallylamine but since the molecular sieve has a much greater affinity for water than for other materials as is well known, the water after being adsorbed to some minimum extent then tends to replace the nitrogen compound on the sieve. Since the presence of water produces chlorides and since water tends to replace the nitrogen compound on the sieve, especially under conditions of relatively high humidity, the presence of water in the system releases diallylamine to react with excess chloride.

Moreover, the molecular sieve besides being a water scavanger is also a hydrochloric acid scavanger. By adsorbing hydrochloric acid, it shifts the equilibrium in reaction No. 3 described herein above to the left thereby forming in the case of diallylamine hydrochloride, diallylamine and HCl. As HCl is produced, it is adsorbed until all of the diallylamine hydrochloride is broken down to diallylamine and HCl. It is surprising that the cleavage due to contact with the molecular sieve results in producing HCl since usually such compounds cleave or break at the bond between the H and Cl of the HCl portion of the molecule.

In addition, since nitrogen compound such as in the form of a chloride complex is removed from prior systems which do not employ a molecular sieve, the prior systems must periodically be replenished with newly added nitrogen compound. However, the present invention surprisingly results in the regeneration of the nitrogen compound and reintroduction of it into the solution when released from the molecular sieve. Accordingly, the present invention makes it possible to maintain the desired level of nitrogen compound in the system without the necessity of replenishing the system with additional fresh nitrogen compound. Of course, if desired fresh nitrogen compound can be added in the practice of the present invention.

According to the present invention, the compositions contain about 10 to about 150 ppm of the nitrogen compound and preferably about 50 to 100 ppm. This is a significant improvement over the use of nitrogen compound such as the diallylamine in prior systems since such required about 200 to about 300 ppm.

The present invention makes it possible to provide compositions after contact with the molecular sieve which contain less than 5 ppm and preferably less than about 1 ppm of chloride and less than 50 ppm and preferably less than 10 ppm of water. Without the use of the molecular sieve, prior processes resulted in about 2 to about 5 ppm of chloride and up to about 130 ppm of water and never below about 20 ppm of water.

In addition, prior processes which merely employed the nitrogen compound required a distillation in order to remove as much of the water as possible. However, the present invention does not require the use of a distillation step but can use such, if so desired, prior to adding the nitrogen compound.

The present invention can be practiced by contacting the composition with a fixed bed of the molecular sieve and at the present time is carried out by flowing the composition up through the bottom of the molecular sieve in a suitable vessel and out the top of the vessel. The vessel for a typical 1892.5 l to 3028 l treatment system contains about 68 kg of molecular sieve as a column about 4 feet high and 2 feet in diameter. The flow rate of the composition is about 4.54±0.76 l per minute and at present time the molecular sieve is regenerated about every eight hours. However, it is believed that once a week would be sufficient for regenerating the molecular sieve. A typical system for treating about 56.78 l of liquid employs about 700 grams of molecular sieve with a flow rate of about 2.84—3.785l/min. The molecular sieve is present as a column about 25.4 cm high and about 10.2 cm in diameter.

Although the acid resistant molecular sieves are preferred according to the present invention, because of the presence of chloride in the system, such is not entirely necessary in view of the small amount of

chloride, the detrimental effect on the molecular sieve using those that are not specifically designated as acid resistant is minimal.

The compositions processed according to the present invention are generally at temperatures of about normal room temperature to about 121°C, with the lower temperatures being preferred since at the lower temperatures, increased amounts of water are adsorbed by the molecular sieve.

The compositions treated according to the present invention are preferably substantially, if not completely free of any saturated chlorinated hydrocarbons.

The following non limiting example is hereby presented to further illustrate the present invention.

Example

About two liters of perchloroethylene containing diallylamine are added to a four neck flask having a thermometer inserted through one neck, connected to a condenser via another neck and being in contact with a heating element. The perchloroethylene is refluxed for about one hour at 100—121°C. The perchloroethylene is then circulated by use of a pump out of the flask via a third neck and through an empty column and then back into the flask via the fourth neck. The perchloroethylene is circulated through conduits which are connected to two of the necks of the flask fitted with stoppers containing apertures for receiving the conduits. The column is then filled with about 50 grams of molecular sieve, Lindy type 4A, 0.32 cm pellets and the perchloroethylene is then recirculated for another hour. The samples for two different runs are analyzed both before and after the presence of the molecular sieve for water, diallylamine, and chloride. The results are produced herein:

Run 1
Without molecular sieve
After reflux and circulation

| | |
|---|---|
| $H_2O$ | 343 ppm |
| diallylamine | 20 ppm |
| $Cl^-$ | 3.2 ppm |

With molecular sieve

| | |
|---|---|
| $H_2O$ | 22 ppm |
| diallyalamine | 74 ppm |
| $Cl^-$ | 1.7 ppm |

Run 2
Without molecular sieve
After reflux and circulation

| | |
|---|---|
| $H_2O$ | 22 ppm |
| diallylamine | 248 ppm |
| $Cl^-$ | <1 ppm |

With molecular sieve

| | |
|---|---|
| $H_2O$ | 5 ppm |
| diallyalamine | 158 ppm |
| $Cl^-$ | <1 ppm |

The above example clearly shows the effectiveness of the molecular sieve on reducing the chloride concentration and increasing the diallylamine concentration after sufficient diallylamine hydrochloride has been broken down into diallylamine and HCl and the HCl absorbed by the molecular sieve.

**Claims**

1. Process for reducing the concentration of chloride in a system containing ethylenically unsaturated chlorinated hydrocarbon, water and HCl omprising the following steps:

a) providing in said system a nitrogen containing compound having a pair of unshared electrons which are capable of forming a dative covalent bond, thereby

b) forming a complex between HCl and said nitrogen containing compound in said system;

c) contacting said system, subsequent to the forming of said complex, with a molecular sieve where, upon contact therewith said complex is broken down into HCl and said nitrogen containing compound, thereby

d) regenerating said nitrogen containing compound and realising it back into said system, and

e) reducing the HCl concentration in said system to less then 5 ppm through adsorption by the molecular sieve.

2. Process of claim 1 wherein said hydrocarbon includes perchloroethylene.

3. Process of claim 1 wherein said nitrogen containing compound is a primary or secondary amine.

4. Process of claim 1 wherein said nitrogen containing compound is diallylamine.

5. Process of claim 1 wherein said nitrogen containing compound has a molecular weight up to about 500.

6. Process of claim 1 wherein said molecular sieve has an effective pore size of about 0.3 to about 1.0 nm.

7. Process of claim 1 wherein said molecular sieve is of the type A or the type X.

8. Process of claim 1 wherein said molecular sieve is an acid resistant molecular sieve.

9. Process of claim 1 wherein said molecular sieve has an effective pore size of 0.4 or 0.5 nm.

10. Process of claim 1 wherein said nitrogen containing compound forms a complex with HCl, said complex is broken down into HCl and said nitrogen compound upon contact with said molecular sieve thereby regenerating said nitrogen compound, said nitrogen compound is released from said sieve and reintroduced into said system.

**Patentansprüche**

1. Verfahren zum Verringern der Chloridkonzentration in einem System, das einem äthylenisch ungesättigten chlorierten Kohlenwasserstoff, Wasser und HCl enthält, das folgende Schritte umfaßt:

a) Bereitstellen einer stickstoffhaltigen Verbindung mit einem Paar nichtanteiliger, zur Ausbildung einer semi-polaren Bundung befähigter Elektronen in dem System, dadurch

b) Ausbilden eines Komplexes zwischen HCl und der stickstoffhaltigen Verbindung im System,

c) Kontaktieren des System, nach der Komplexbildung, mit einem Molekularsieb zur Aufspaltung des Komplexes in HCl und die stickstoffhaltige Verbindung, dadurch

d) Regenerieren der stickstoffhaltigen Verbindung und Wiedereinführen in das System und

e) Reduzieren der HCl-Konzentration in dem System durch Adsorption an dem Molekularsieb weniger als 5 ppm.

2. Verfahren nach Anspruch 1, dadurch gekennzeichnet, daß der Kohlenwasserstoff Perchloräthylen umfaßt.

3. Verfahren nach Anspruch 1, dadurch gekennzeichnet, daß die stickstoffhaltige Verbindung ein primäres oder sekundäres Amin ist.

4. Verfahren nach Anspruch 1, dadurch gekennzeichnet, daß die stickstoffhaltige Verbindung Diallylamin ist.

5. Verfahren nach Anspruch 1, dadurch gekennzeichnet, daß die stickstoffhaltige Verbindung ein Molekulargewicht bis zu etwa 500 aufweist.

6. Verfahren nach Anspruch 1, dadurch gekennzeichnet, daß das Molekulargewicht eine effektive Porengröße von etwa 0,3 bis etwa 1,0 nm aufweist.

7. Verfahren nach Anspruch 1, dadurch gekennzeichnet, daß das Molekularsieb von A- oder X-Typ ist.

8. Verfahren nach Anspruch 1, dadurch gekennzeichnet, daß das Molekularsieb säurebeständig ist.

9. Verfahren nach Anspruch 1, dadurch gekennzeichnet, daß das Molekularsieb eine effektive Porengröße von etwa 0,4 bis 0,5 nm aufweist.

10. Verfahren nach Anspruch 1, dadurch gekennzeichnet, daß die stickstoffhaltige Verbindung mit HCl einen Komplex bildet, der bei Kontakt mit dem Molekularsieb in HCl und die Stickstoffverbindung audgespalten wird, wodurch die Stickstoffverbindung regeniert, von dem Sieb freigegeben und in das System wieder eingeführt wird.

**Revendications**

1. Procéde pour réduire la concentration en chlorure dans un système qui contient un hydrocarbure chloré à insaturation éthylénique, de l'eau et HCl, comprenant les stades suivants:

a) la fourniture d'un composé azoté ayant une paire d'électrons non partagés pour la formation d'une liaison semi-polaire des électrons concernés dans le système, et par suite

b) la formation d'un complex entre HCl at le composé azoté dans le système,

c) la mise en contact du système, après la formation du complexe, avec un tamis moléculaire pour une décomposition du complexe en HCl et ledit composé azoté, et par suite

d) la régénération de composé azoté et la réintroduction dans le système et

e) la réduction de la concentration en HCl dans le système par adsorption sur ledit tamis moléculaire à moins de 5 ppm

2. Procédé selon la revendication 1, dans lequel ledit hydrocarbue comprend du perchloroéthlène.

3. Procédé selon la revendication 1, dans lequel ledit composé contenant de l'azote est une amine primaire ou secondaire.

4. Procédé selon la revendication 1, dans lequel ledit composé contenant de l'azote est une diallylamine.

5. Procédé selon la revendication 1, dans lequel composé contenant a un poids moléculaire pouvant atteindre environ 500.

6. Procédé selon la revendication 1, dans lequel ledit tamis moléculaire a des pores dont la dimension efficace est d'ordre de 0,3 à 1,0 nm.

7. Procédé selon la revendication 1, dans lequel ledit tamis moléculaire est du A ou du type X.

8. Procédé selon la revendication 1, dans lequel ledit tamis moléculaire est un tamis moléculaire résistant à l'acide.

9. Procédé selon la revendication 1, dans lequel ledit tamis moléculaire a des pores dont la dimension efficace est 0,4 ou 0,5 nm.

10. Procédé selon la revendication 1, dans lequel le composé contenant de l'azote forme un complex avec HCl, ledit complexe se décompose pour donner HCl et ledit composé d'azote par contact avec ledit tamis moléculaire afin de réengendrer ledit composé d'azote, ledit composé d'azote est libéré dudit tamis et réintroduit dans ledit système.